# EUROPEAN PATENT SPECIFICATION

(11) **EP 0 583 979 B1**
(45) Date of publication and mention of the grant of the patent: **05.11.1997**
(21) Application number: 93306502.1
(22) Date of filing: 18.08.1993
(51) Int. Cl.: C07C 69/013, C07C 49/643, C07C 49/683, C07C 67/03, C12P 7/40, C07C 45/67, C07C 49/657

(54) **Cyclohexene and cyclohexenone derivatives and process for production of cyclohexene derivatives**
Cyclohexen und Cyclohexenonderivate und Verfahren zum Herstellen von Cyclohexenderivaten
Dérivés du cyclohexène et de la cyclohexénone et procédé de production de dérivés du cyclohexène

(30) Priority: 19.08.1992 JP 290684/92
(43) Date of publication of application: 23.02.1994
(73) Proprietor: CHISSO CORPORATION, Osaka-shi Osaka 530 (JP)
(72) Inventor: Takano, Seiichi, Sendai-shi Miyagi-ken (JP); Ogasawara, Kunio, Sendai-shi Miyagi-ken (JP)
(74) Representative: W.P. THOMPSON & CO.

(56) References cited:
- JOURNAL OF THE CHEMICAL SOCIETY, CHEMICAL COMMUNICATIONS, no. 9, 7 May 1993, Letchworth, GB, pages 788 - 789; S. TAKANO et al, "Enantiocontrolled synthesis of optically pure 5-trimethylsilyl-and 5-tributylstannyl-cyclohex-2-enones"
- BULLETIN OF THE CHEMICAL SOCIETY OF JAPAN, vol. 63, no. 2, February 1990, Tokyo, JP, pages 407 - 411; M. ASAOKA et al, "A new enantioselective approach to the bicyclo[4.4.0.]decane and bicyclo[4.3.0]nonane systems"

## Description

The present invention relates to optically active cyclohexene derivatives, processes of production of optically active cyclohexenone derivtives, which include compounds represented by the following formulae, useful as a starting material for synthesizing natural products, and intermediates used for the above production.

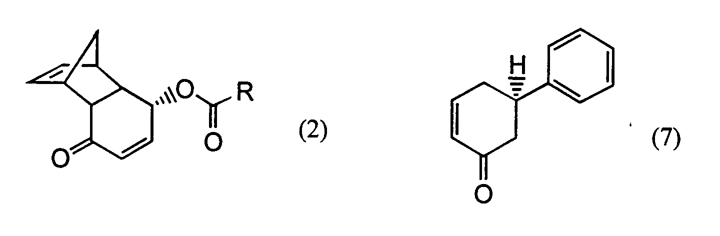

The optically active cyclohexenone derivative represented by the formula (7) is a very useful compound as an intermediate for synthesizing natural materials (for example, "Total Synthesis of Natural Products", ApSimon edit, vol. 2, 1973 and vol. 5, 1983, and J. d'Angelo, Tetrahedron, 32, 2979 (1976)). Takei et al. synthesized an optically active cyclohexenone derivative (7) by using optically active 5-trimethylsilylcyclohexen-2-one as a starting material (J. Chem. Soc., Chem. Commun., 1988, 430).

Takei et al. have reported further syntheses of derivative (7), using optically active 5-trimethylsilylcyclohexen-1-one as a starting material, in Bull. Chem. Soc. Jpn., 63, 407-411 (1990). This document describes the synthesis of derivative (7) from a racemate of 5-trimethylsilyl-2-cyclohexen-1-one via a Diels-Alder adduct with cyclopentadiene followed by oxidative desilylation to form a racemate of an optically active derivative represented by the formula: 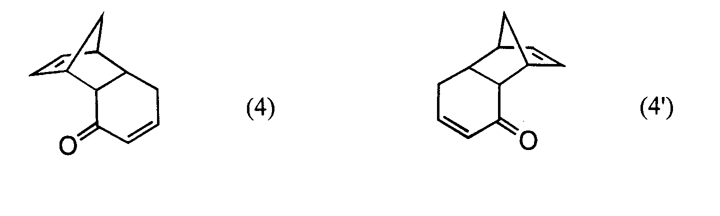 Conjugate reduction with K-selectride followed by the copper (I) catalysed 1,4-addition of phenylmagnesium bromide to the (+)-enantiomer is reported to yield the (+)-enantiomer represented by the following formula: 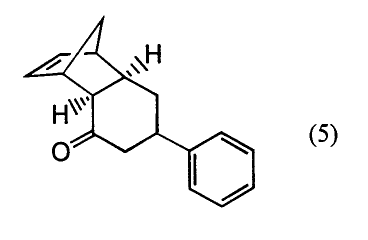 A retro Diels-Alder reactor catalysed by BF₃ etherate then yields the (+)- enantiomer of derivative (7).

However, optically active 5-trimethylsilylcyclohexen-2-one is obtained by repeated recrystallization of a diastereomer salt synthesized from a racemate of the starting material with cinchonidine and toluenethiol. The method is not for practical use because it has many steps and a low yield of 3% (Tetrahedron, Lett., 28, 5669 (1987).

The optically active cyclohexeneone derivative represented by the formula (2) is a starting material for synthesizing eurypoxide B, a metabolite of the fungus Eurypa lata. The racemic total synthesis of eutypoxide B has been reported by Tabacci and co-workers and employs a Diels-Alder reaction as the key step (Helv. Chim. Acta, 75, 276 (1992)). However, the key reaction directed diasterofacial selection incorrectly to furnish the diastereomeric mixture containing the desired epimer only in a ratio which could be fortuitously separated in a later stage.

Thus, prior to the present invention, there is no efficient method for producing optically active cyclohexenone derivatives.

Considering the above, the inventors of the present invention have earnestly studied to attain this object and to efficiently obtain optically active cyclohexenone derivatives having high purity as intermediates for synthesizing natural compounds, so that they have found optically active cyclohexene derivatives useful as intermediates and the optically active cyclohexenone derivatives, and the method for producing efficiently the compounds has been established.

The present invention can provide a process in which an optically active cyclohexenone derivative is obtained by a process comprising using as a starting material a cyclohexene diol represented by the formula: 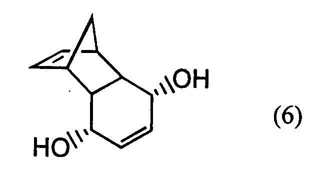 reacting the cyclohexene diol with an ester in the presence of lipase by transesterification selectively in a position to obtain an optically active cyclohexene derivative represented by the general formula: 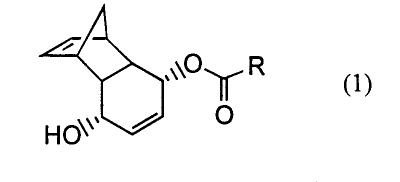 or 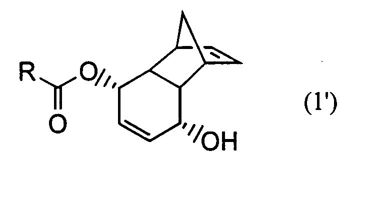 wherein R is alkyl, then oxidizing the hydroxy group of the derivative to obtain an optically active cyclohexenone derivative represented by the formula: 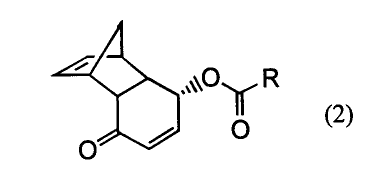 or 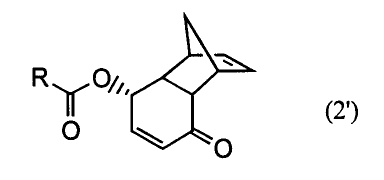 wherein R is alkyl, and reacting the resultant derivative by deacyloxylation to obtain an optically active cyclohexenone derivative represented by the formula: 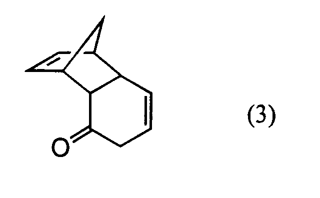 or 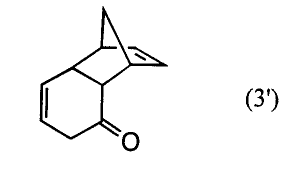 Then, the last compound is isomerized to obtain an optically active cyclohexenone derivative represented by the formula: 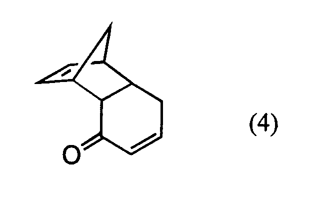 or 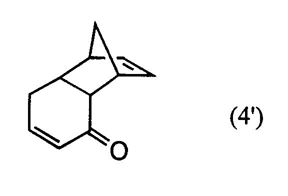 reacting the resultant compound with a Grignard reagent to obtain an optically active derivative represented by the formula: 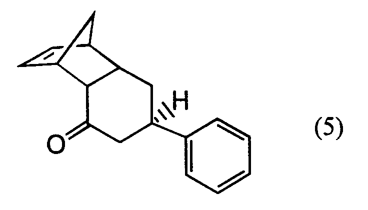 or 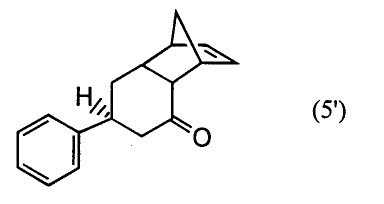 and reacting the resultant compound by a retro Diels-Alder reaction to obtain an optically active cyclohexenone derivative represented by the formula: 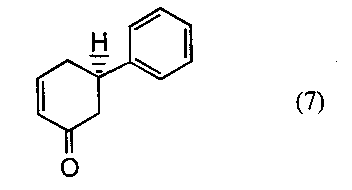 or 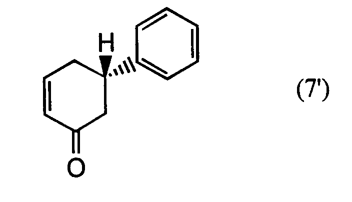 The reaction steps of the process of the present invention are exemplified in the following: 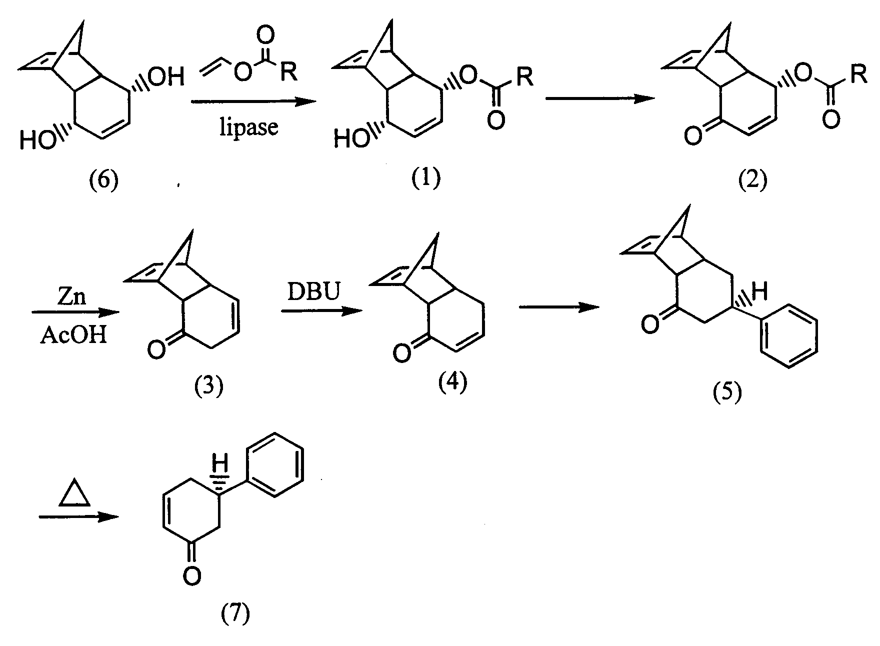 The cyclohexene diol derivative (6) of the starting material in the present invention can easily be obtained by reduction of a carbonyl group of a cyclohexenone derivative, which can be obtained by a common Diels-Alder reaction of cylcopentadiene and hydroquinone, in the presence of a reducing agent such as lithium aluminum hydride.

The resultant hexenediol derivative (6) may be reacted by transesterification with a specified ester in the presence of lipase. Otherwise, the derivative (6) may be reacted by a conventional method and the diacetate obtained hydrolyzed in the presence of lipase. Then, the optically active cyclohexene derivative (1) or (1') of the present invention can be obtained. In the case of the transesterification reaction, fatty acid vinyl esters, triglycerides, acid anhydrides and several kinds of esters are examples of the specified ester. Fatty acid vinyl esters are preferably used. Any lipase having a catalytic effect in the reaction can be used. In particular, commercially available lipase PS (manufactured by Amano Pharmaceutical Co., Ltd.) derived from a Pseudomonus genus is preferred. Any solvent by which the reaction is not inhibited can be used, or the reaction can be conducted in the presence of the substrate alone and without solvent. Preferably, acetonitrile is used as a reaction solvent.

When the cyclohexene derivative (1) is reacted with an oxidising agent such as manganese dioxide by a common oxidation reaction, a hydroxyl group is changed into a carbonyl group to obtain the cyclohexene derivative represented by the formula (2).

Then the resultant derivative can be treated with ultrasonic waves in the presence of zinc to deacyloxylate by a common method, and the cylcohexenone derivative represented by the formula (3) obtained.

Further, the resultant compound is isomerized by any method to obtain the cyclohexenone derivative represented by the formula (4).

The compound (4) may be reacted with a Grignard reagent such as phenyl magnesium bromide by a nucleophilic raction (Takei et al., Tetrahedron Lett., 28, 5669 (1987)), and the cylcohexenone derivative represented by the formula (5) obtained.

The compound can be changed into the optically active cylcohexenone derivative represented by the formula (7) useful as an intermediate of natural compounds by a general retro Diels-Alder reaction.

The alkyl groups R and R' are preferably C₁ to C₃₅ alkyl groups, more preferably C₁ to C₂₂ alkyl groups.

The present invention can provide the following merits.
1. Optically active cyclohexene derivatives useful for natural material synethesis, which are represented by the above formulas (1) or (1') - (4) or (4') and (7) or (7'), are obtained efficiently and the derivatives have high purity.
2. Each intermediate represented by the above formulas (1) or (1') - (5) or (5') and (7) or (7') can be changed into useful chiral elements.
3. The cyclohexene derivative represented by the above formula (1) or (1') is a meso compound, so that it acts as two antipodes by distinction between a hydroxy group and an acyloxy group. Accordingly, the compound (1) or (1') can be prepared from the same compound (6), and the compound (2) or (2') and the like can be prepared similarly.

The following examples illustrate this invention more specifically, but these will not always be precise in practical applications.

### Example 1

In an acetonitrile solution of a hexenediol derivative (6) (100 mg, 0.56 mmol) and vinyl acetate (0.3 ml, 3.36 mmol), lipase PS (100 mg) was suspended, and the mixture was stirred for two weeks at room temperature. After the lipase was filtered away, the filtrate was concentrated under reduced pressure, and the residue was purified with a chromatograph over silica gel to obtain a diacetate (5 mg, 3.4%), a monoacetate (1) (101 mg, 89%) and 15 mg of unreacted materials. The specific rotation of the monoacetate was [α]_{D}²⁹ 63.1° (c2.20, CHCl₃). Further, the other values of physical properties were as follows:
IR (neat) 1737 cm⁻¹
¹H-NMR (90 MHz), CDCl₃
   1.20-1.40(m, 2H), 1.73-2.25(br, 1H), 2.08(s, 3H), 2.71-3.18(m, 4H), 4.39-4.48(m, 1H), 5.19-5.57(m, 3H), 5.74-5.93(m, 2H).

### Example 2

A dichloromethane solution (5 ml) of the monoacetate (1) (600.3 mg, 2.72 mmol) was added dropwise to a suspended dichloromethane solution (10 ml) of manganese dioxide (3.56 g, 40.9 mmol) on ice cooling, and the mixture was stirred for 30 minutes at the same temperature. Unreacted materials were filtered off, the filtrate was concentrated under reduced pressure, and the residue was chromatograped over silica gel to obtain a ketone (2) (491 mg, 82%). The specific rotation was [α]_{D}²⁹ -219.9 ° (c1.29, CHCl₃). Further, the value of elemental analysis was as follows:

| | | |
|---|---|---|
| Calculated | C:71.54, | H:6.47 |
| Found | C:71.43, | H:6.47 |

The other values of physical properties were as follows:
IR (neat) 1741, 1669 cm⁻¹
¹H-NMR (90 MHz), CDCl₃
   1.23-1.51(m, 2H), 2.17(s, 3H), 2.89-3.48(m, 4H), 5.60-5.93(m, 3H), 6.11(dd, 1H, J=5.4Hz, 2.7Hz), 6.45(ddd, 1H, J=10.2, 2.4, 1.0Hz)

### Example 3

In a mixed solution of the ketone (2) (97 mg, 0.44 mmol) in ethanol-acetic acid (2.1 ml-0.7 ml), 150 mg of zinc powder was suspended, and the mixture was irradiated with ultrasonic waves at room temperature for 3.5 hours. Insoluble materials were filtered off, the filtrate was concentrated under reduced pressure, and the residue was extracted with ether. The ether layer was washed with 5% aqueous solution of sodium bicarbonate and with water, and then the layer was dried over magnesium sulfate. The solvent was distilled away under reduced pressure, and the residue was chromatographed over silica gel to obtain the compound represented by the formula (3) (42 mg, 43%). The values of physical properties were as follows:
IR (neat) 1701 cm⁻¹
¹H-NMR (90 MHz), CDCl₃
   1.31-1.55(m, 2H), 2.31-2.74(m, 2H), 2.80-3.22(m, 3H), 2.80-3.22(m, 3H), 3.28-3.46(m, 1H), 5.43-5.64(m, 1H), 5.72-5.89(m, 1H), 5.98-6.28(m, 2H)

### Example 4

A β, γ-unsaturated ketone (3) (42 mg, 0.26 mmol) and DBU (0.04 ml, 0.26 mmol) were dissolved in benzene (2 ml), and the mixture was stirred overnight at room temperature. Benzene was distilled away under reduced pressure, the residue was chromatographed over silica gel, and a α, β-unsaturated ketone (4) (10 mg, 24%) was obtained. The values of physical properties were as follows:
IR (neat) 1662 cm⁻¹
¹H-NMR (90 MHz), CDCl₃
   1.24-1.52(m, 2H), 1.84-2.26(m, 1H), 2.39-3.10(m, 4H), 3.30-3.48(m, 1H), 5.78-5.97(m, 1H), 6.04-6.23(m, 2H), 6.57-6.79(m, 1H)

### Example 5

An α, β-unsaturated ketone (4) (10 mg, 24%) was dissolved in 10 ml of THF. To the solution, phenyl magnesium bromide (0.075 mmol, 10 ml of a THF solution) previously prepared was added dropwise on ice cooling. After stirring for four hours, the reaction mixture was injected into a saturated solution of ammonium chloride on ice cooling. After the mixture was extracted with ether, the ether layer was washed with saturated sodium chloride solution. The ether layer was dried over magnesium sulfate, then ether was distilled away under reduced pressure, and a residue was obtained. The residue was purified by silica gel chromatography to obtain an optically active cyclohexanone derivative (5) (9 mg, 62%). Further, the compound in 1 ml of a diphenyl ether solution was heated and refluxed for 1.5 hours. The mixture solution was chromatographed over silica gel to obtain an optically active cyclohexenone derivative represented by the formula (7) (2.5 mg, 90%). The specific rotation was [α]_{D}³¹ -47.9° (c0.52, CHCl₃), (a literature value ; [α]_{D}²³ -46.4° (c5.0, CHCl₃), and the result fits in with the literature value.

### Example 6 Synthesis of eutypoxide B (Application example)

### Step 1

Vinyl magnesium bromide (2.1 ml, 1M THF sol.) was added to 4 ml of a THF solution of copper (I) bromide-dimethyl sulfide complex (7 mg, 0.03 mmol) and HMPA (0.25 ml, 1.4 mmol) at -78 °C and the mixture was stirred for 10 minutes. A mixture of the enone (153 mg, 0.70 mmol) obtained by Example 5 and trimethylsilyl chloride (0.36 ml, 2.84 mmol) dissolved in 2 ml of THF was added dropwise to the above mixture and stirred for one hour at -78 °C. Then, 3 ml of 5% hydrochloric acid was added. After rising to room temperature the mixture was stirred for 30 minutes. The reaction solution was diluted with ether, and washed with water, with saturated sodium bicarbonate solution and then with saturated sodium chloride solution. The ether layer was dried over magnesium sulfate, then ether was distilled away under reduced pressure, and the residue was obtained. The residue was purified by silica gel chromatography to obtain a ketone (140 mg, 81%) of colorless crystals.
m.p. 73-74°C
[α]_{D}³² +20.9° (c 0.94, CHCl₃)
IR (nujol) 1723, 1687 cm⁻¹
¹H-NMR (300 MHz), CDCl₃; 1.29(br. d, J=8.4 Hz, 1H), 1.41(br. d, J=8.6 Hz, 1H), 2.11(s, 3H), 2.11-2.33(m, 2H), 2.53-2.61(m, 1H), 2.90-2.96(m, 2H), 3.05-3.12(m, 1H), 3.38(br. s, 1H), 4.98-5.07(m, 2H), 5.18(dd, J=10.7, 8.1 Hz, 1H), 5.60(ddd, J=17.2, 10.6, 6.3 Hz, 1H), 6.14-6.21(m, 2H)
MS m/e 246(M⁺), 66(100%)
HRMS calcd for C₁₅H₁₈O₃ 246.1256, Found 246.1253
Anal. calcd for C₁₅H₁₈O₃: C73.15; H 7.37 Found C 73.14; H 7.34.

### Step 2

Sodium borohydride (94 mg, 2.5 mmol) was added to 3 ml of a methanol solution of the ketone (305 mg, 1.24 mmol) obtained by Step 1 on ice cooling, and the mixture was stirred at the same temperature for 30 minutes. After rising to room temperature, potassium carbonate (300 mg, 2.17 mmol) was added, and the mixture was stirred for 12 hours. The mixture was diluted with dichloromethane and washed with saturated sodium chloride solution. The dichloromethane layer was dried over magnesium sulfate, then the organic solvent was distilled away under reduced pressure, and the residue was chromatographed over silica gel to obtain a diol (235 mg, 92%).
m.p. 132-133°C
[α]_{D}²⁷ +72.2° (c 1.16, MeOH)
IR (nujol) 3235 cm⁻¹
¹H-NMR (300 MHz), CDCl₃; 1.33(d, J=8.1 Hz, 1H), 1.45-1.54(m, 2H), 1.85(ddd, J=14.7, 10.2, 7.3Hz, 1H), 2.01(br. d. J=5.1Hz, 1H), 2.30-2.36(m, 1H), 2.45-2.54(m, 2H), 2.62(ddd, J=11.0, 5.1, 3.3Hz, 1H), 2.94-2.98(m, 1H), 3.76-3.82(m, 1H), 4.14-4.18(m, 1H), 5.00-5.06(m, 2H), 5.71(ddd, J=16.9, 9.9, 8.4 Hz, 1H), 6.18-6.25(m, 2H)
MS m/e 206(M⁺), 66(100%)
HRMS calcd for C₁₃H₁₈O₂ 206.1307, Found 206.1313
Anal. calcd for C₁₃H₁₈O₂: C 75.69; H 8.8; Found C 75.69; H 8.8.

### Step 3

Four ml of a diphenyl ether solution of the diol (100 mg, 0.49 mmol) obtained by Step 2 was refluxed for 45 minutes. The reaction solution was chromatographed over silica gel to obtain a retro D-A product (50 mg, 73%).
[α]_{D}³⁰ -33.2° (c 0.43, MeOH)
IR (film) 3328 cm⁻¹
¹H-NMR (300 MHz), CDCl₃; 1.57(ddd, J=14.3, 12.1, 4.4 Hz, 1H), 1.72(dt, J=13.5, 3.0 Hz, 1H), 2.26-2.37(m, 1H), 3.71(d, J=8.5 Hz, 1H), 4.01-4.04(m, 1H), 4.93-5.08(m, 2H), 5.69(br. s, 2H), 5.79(ddd, J=14.5, 10.6, 7.3 Hz, 1H)
MS m/e 122(M⁺-18), 86(100%)
HRMS calcd for C₈H₁₀O 122.0732, Found 122.0725

### Step 4

Imidazole (50 mg, 0.73 mmol) and t-butyldimethylsilyl chloride (81 mg, 0.54 mmol) were added to 0.5 ml of a DMF solution of retro D-A product (25 mg, 0.18 mmol) on ice cooling, and the mixture was stirred for four hours at room temperature. Ether was added to the reaction solution, and the solution was washed with saturated sodium chloride and dried over magnesium sulfate. The solvent was distilled away under reduced pressure, and the residue was chromatographed over silica gel to obtain t-butyldimethylsilyl ether (62 mg, 94%).
[α]_{D}²⁶ -9.6° (c 1.03, CHCl₃)
IR (film) 1642 cm⁻¹
¹H-NMR (90 MHz), CDCl₃; 0.06(s, 12H), 0.90(s, 18H), 1.42-1.91(m, 2H), 2.34-2.58(m, 1H), 3.86(d, J=8.0 Hz, 1H), 4.10-4.22(m, 1H), 4,95-5.19(m, 2H), 5.65(d, J=1.2 Hz, 2H), 5.65-6.12(m, 1H)
MS m/e 368(M⁺), 147(100%)
HRMS calcd for C₁₆H₃₄O₂Si₂ 314.2097 (M⁺-54),
Found 314.2110

### Step 5

m-Chloroperbenzoic acid (205 mg, 0.83 mmol) and 4,4'-Thiobis (6-tert-butyl-m-cresol)(10 mg, 0.028 mmol) were added to 5 ml of a dichloroethane solution of t-butyldimethylsilyl ether (102 mg, 0.28 mmol), and the mixture was refluxed for 5 minutes. The mixture was diluted with dichloromethane, and washed with saturated sodium bicarbonate solution and then with saturated sodium chloride solution. The organic layer was dried over magnesium sulfate, and the solvent was distilled away under reduced pressure. The residue was chromatographed over silica gel to obtain a diepoxide (82 mg, 74%).
¹H-NMR (90 MHz), CDCl₃; 0.10(s, 8H), 0.17(s, 4H), 0.90, 0.93, 0.94(3s, 18H), 1.15-1.68(m, 3H), 2.35-2.79 (m, 3H), 3.07(br. s, 2H) 3.73(d, J=8.6 Hz, 1H), 4.18-4.29(m, 1H)
MS m/e 343(M⁺-^{t}Bu), 147(100%)
HRMS calcd for C₁₆H₃₁O₂Si₂ 343.1761(M⁺-^{t}Bu), Found 343.1754

### Step 6

Isopropenylmagnesium bromide (2 ml, 0.6M-THF sol., 1.2 mmol) was added to 2 ml of a THF solution of diepoxide (82 mg, 0.21 mmol) and CuI (5 mg, 0.03 mmol) at -20 °C, and the mixture was stirred at -15 °C for 4 hours. A saturated solution of ammonium chloride was added to the reaction solution, and the solution was extracted with ether. The ether layer was washed with water, with saturated sodium bicarbonate solution and with saturated sodium chloride solution. The ether layer was dried over magnesium sulfate. The solvent was distilled away under reduced pressure and a crude product (111 mg) was obtained. The product was dissolved in 3 ml of dichloromethane, pyridinium chlorochromate (90 mg, 0.41 mmol) was added, and the mixture was stirred for 10 hours at room temperature. 3g of silica gel was added to the reaction solution, and the solution was filtered with cerite. The solvent was distilled away from the filtrate and the residue was chromatographed over silica gel to obtain a ketone (60 mg, 89%).
m.p. 78-79 °C
[α]_{D}²⁹ +18.3° (c 0.71, CHCl₃)
IR (nujol) 1706 cm⁻¹
¹H-NMR (300 MHz), CDCl₃; 0.04 (s, 3H), 0.11(s, 3H), 0.12 (s, 3H), 0.14(s, 3H), 0.89(s, 9H), 0.94(s, 9H), 1.45-1.56(m, 2H), 1.74(s, 3H), 2.98(t. d, J=9.5, 5.4 Hz, 1H) 3.04-3.08(m, 2H), 3.09(d, J=15.0 Hz, 1H), 3.17(d, J=15.0 Hz, 1H), 4.12(d, J=9.5 Hz, 1H), 4.21-4.25(m, 1H), 4.81(s, 1H), 4.94(s, 1H),
MS m/e 425(M⁺-CH₃), 73(100%)
HRMS calcd for C₂₂H₄₁O₄Si₂ 425.2543(M⁺-CH₃),
Found 425.2549
Anal. calcd for C₂₂H₄₁O₄Si₂: C 62.68; H 10.06;
Found C 62.82; H 10.06.

### Step 7

Bu₄NF (0.20 ml, 1M-THF sol. 0.2 mmol) was added to the enome (20 mg, 0.045 mmol) obtained by Step 6, and the mixture was stirred for one hour. After saturated sodium chloride solution was added, the mixture was extracted with ethyl acetate, and the organic layer was dried over magnesium sulfate. The solvent was distilled away under reduced pressure, and the residue was chromatographed over silica gel to obtain eutypoxide B (6.1 mg, 63%).
[α]_{D}²³ -56.6° (c 0.68, CHCl₃)
IR (film) 3414, 1678, 1614 cm⁻¹
¹H-NMR (300 MHz), CDCl₃; 1.50(t. d, J=13.9, 2.9 Hz, 1H), 1.80(t. d, 12.1 Hz, 2.8 Hz, 1H), 1.92(d, J=1.1 Hz, 3H), 2.06(d, J=5.5 Hz, 1H), 2.16(d, J=0.7 Hz, 3H), 2.67(ddd, 12.8, 9.5, 2.5 Hz, 1H), 2.89(d, J=4.4 Hz, 1H), 3.22(br. s, 1H), 3.25(d, J=3.3 Hz, 1H), 4.25(dd, J=4.4, 9.5 Hz, 1H), 4.36-4.42(m, 1H), 6.14(br. s, 1H)
MS m/e 212(M⁺), 83(100%)
HRMS calcd for C₁₁H₁₆O₄ 212.1049(M⁺), Found 212.1000

### Example 8

To 30 ml of a phosphoric acid buffer containing 10% (v/v) acetone, 3,6-diacetyloxytricyclo [6,2,1,0^{2.7}] undeca-4,9-diene (8) (1.31 g, 5.0 mmol) was mixed. 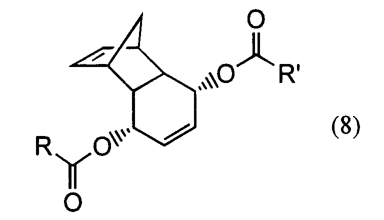 After lipase PS (30 mg) was added, and the mixture was shaken at 27°C. After 24 hours, the reaction solution was filtered with cerite, the filtrate was extracted with ether. The organic layer was dried over magnesium sulfate, and the solvent was distilled away under reduced pressure. The residue was purified with a chromatograph over silica gel to obtain a monoacetate (1') (930 mg, 4.23 mmol, 85%). The specific rotation of the monoacetate was [α]_{D}²⁹ -62.8° (c2.0, CHCl₃). Further, the data of ¹H-NMR fit in with those of the compound (1) of Example 1.

## Claims

1. An optically active cyclohexene derivative represented by the general formula: 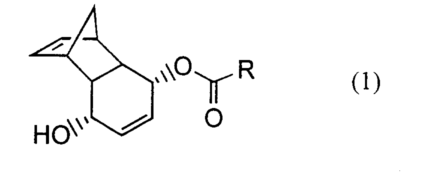 or 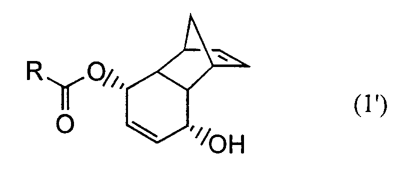 wherein R is alkyl.

2. An optically active cyclohexenone derivative represented by the general formula: 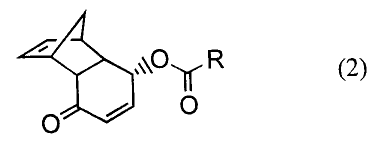 or 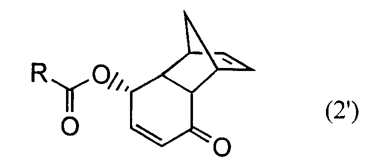 wherein R is alkyl.

3. An optically active cyclohexenone derivative represented by the following formula: 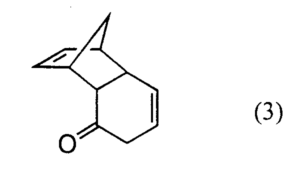 or

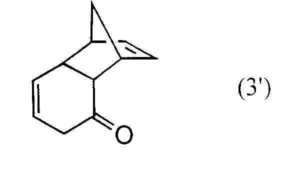

4. A process for producing an optically active cyclohexene derivative represented by the formula (1) or (1') of claim 1 comprising using the cyclohexene diol represented by the following formula as a starting material: 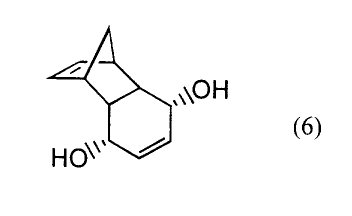 and subjecting the cyclohexene diol to transesterification at a position selectively to obtain the desired optically active cyclohexene derivative.

5. A process according to claim 4, in which the cyclohexene diol is transesterified in the presence of lipase to obtain the optically active derivative.

6. A process according to claim 4, in which the cyclohexene diol is unselectively transesterified and the diester subsequently selectively hydrolyzed in the presence of lipase.

7. A process for producing a desired optically active cyclohexenone derivative represented by the following formula: 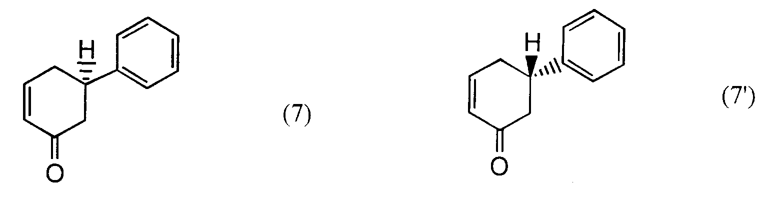 comprising using a first optically active cyclohexenone derivative represented by the formula (2) or (2') of claim, as a starting material, reacting the first derivative by deacyloxylation to obtain a second optically active cyclohexenone derivative represented by the formula (3) or (3') of claim 3, isomerising the second derivative to obtain a third optically active cyclohexenone derivative represented by the formula (4) or (4'), reacting the third derivative with a Grignard reagent to obtain an optically active cyclohexanone derivative representated by the formula (5) or (5'), and reacting the cyclohexanone derivative by a retro Diels-Alder reaction to obtain the desired optically active cyclohexenone derivative.

8. A process for producing an optically active cyclohexene derivative represented by the formula (1) or (1') of claim 1 comprising using a diacyloxy cyclohexene derivative represented by the following formula as a starting material, wherein R and R' are the same or different and each represents an alkyl group: 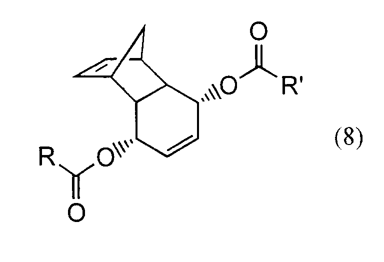 and reacting the diacycloxy cyclohexene derivative in the presence of lipase by stereoselective hydrolysis to obtain the desired optically active cyclohexene derivative.

## Patentansprüche

1. Optisch aktives Cyclohexenderivat, welches durch die allgemeine Formel dargestellt ist: 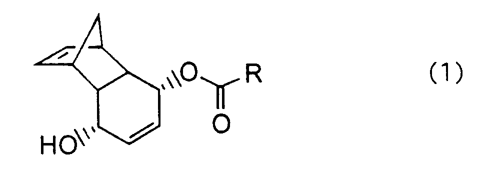 oder 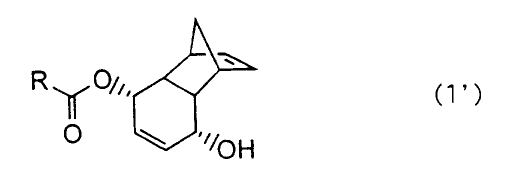 wobei R ein Alkyl ist.

2. Optisch aktives Cyclohexenonderivat, welches durch die allgemeine Formel dargestellt ist: 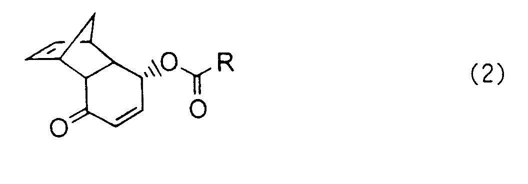 oder 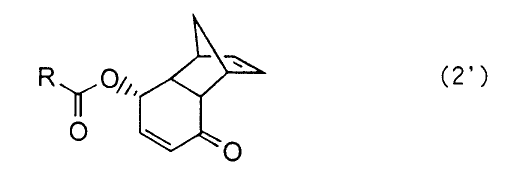 wobei R ein Alkyl ist.

3. Optisch aktives Cyclohexenonderivat, welches durch die folgende Formel dargestellt ist: 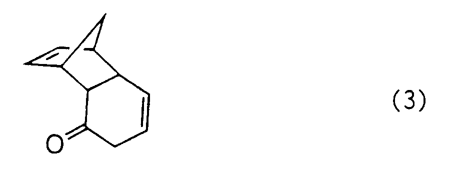 oder

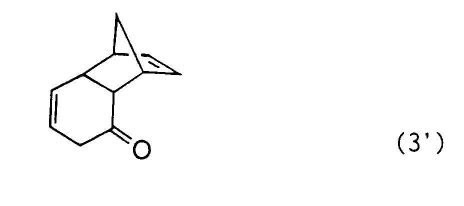

4. Verfahren zur Herstellung eines optisch aktiven Cyclohexenderivats, welches durch die Formel (1) oder (1') des Anspruchs 1 dargestellt ist, wobei das in der folgenden Formel dargestellte Cyclohexendiol als Ausgangsmaterial benutzt wird: 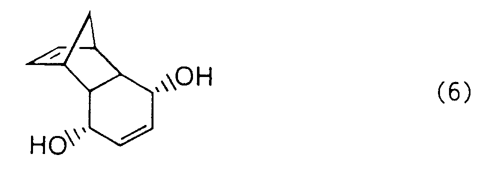 und wobei das Cyclohexendiol in einer Stellung selektiv einer Umesterung unterzogen wird, um das gewünschte optisch aktive Cyclohexenderivat zu gewinnen.

5. Verfahren nach Anspruch 4, wobei das Cyclohexendiol in Gegenwart von Lipase einer Umesterung unterzogen wird, um das optisch aktive Derivat zu gewinnen.

6. Verfahren nach Anspruch 4, wobei das Cyclohexendiol unselektiv einer Umesterung unterzogen wird und wobei das Diester anschließend selektiv in Gegenwart von Lipase hydrolisiert wird.

7. Verfahren zur Herstellung eines gewünschten optisch aktiven Cyclohexenonderivats, welches durch die folgende Formel dargestellt ist 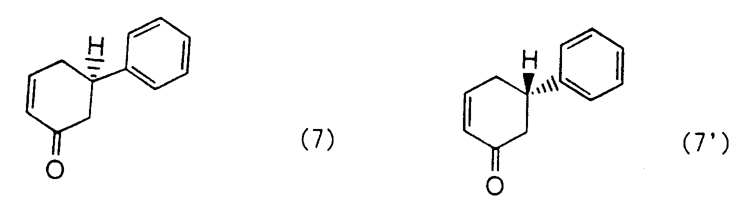 wobei ein erstes optisch aktives Cyclohexenonderivat, welches durch die Formel (2) oder (2') gemäß Anspruch 2 dargestellt ist als Ausgangsmaterial benutzt wird, wobei das erste Derivat durch Entacyloxylierung umgesetzt wird, um ein zweites optisch aktives Cyclohexenonderivat zu gewinnen, welches durch die Formel (3) oder (3') gemäß Anspruch 3 dargestellt ist, wobei das zweite Derivat isomerisiert wird, um ein drittes optisch aktives Cyclohexenonderivat zu gewinnen, welches durch die Formel (4) oder (4') dargestellt ist, wobei das dritte Derivat mittels eines Grignard Reagenz umgesetzt wird, um ein optisch aktives Cyclohexanonderivat zu gewinnen, welches durch die Formel (5) oder (5') dargestellt ist, und wobei das Cyclohexanonderivat mittels einer umgekehrten Diels-Alder Reaktion umgesetzt wird, um das gewünschte optisch aktive Cyclohexenonderivat zu gewinnen.

8. Verfahren zur Herstellung eines optisch aktiven Cyclohexenderivats, welches durch die Formel (1) oder (1') des Anspruchs 1 dargestellt ist, indem ein Diacyloxycyclohexenderivat als Ausgangsmaterial benutzt wird, welches durch die folgende Formel dargestellt ist, wobei R und R' einander gleich oder voneinander verschieden sind und jeweils eine Alkylgruppe darstellen: 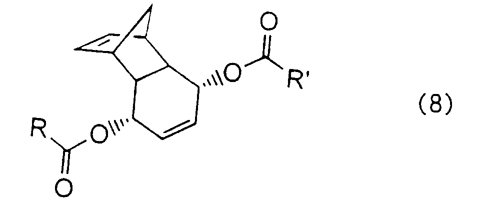 und wobei das Diacyloxycyclohexenderivat in Gegenwart von Lipase mittels einer stereoselectiven Hydrolyse umgesetzt wird, um das gewünschte optisch aktive Cyclohexenderivat zu gewinnen.

## Revendications

1. Un dérivé de cyclohexène optiquement actif représenté par la formule générale : 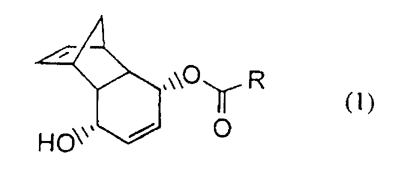 ou 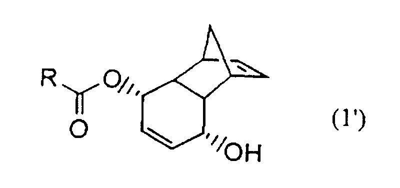 dans laquelle R est alkyle.

2. Un dérivé de cyclohexénone optiquement actif représenté par la formule générale : 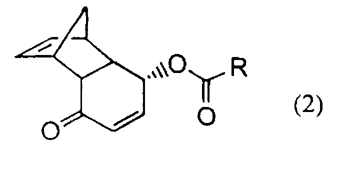 ou 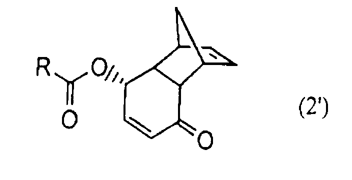 dans laquelle R est alkyle.

3. Un dérivé de cyclohexénone optiquement actif représenté par la formule générale : 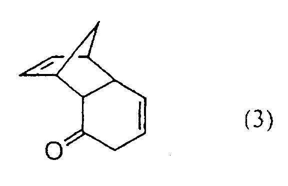 ou

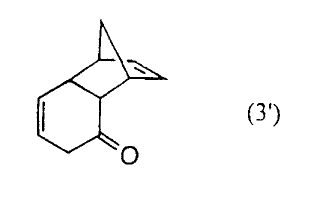

4. Un procédé pour produire un dérivé de cyclohexène optiquement actif représenté par la formule (1) ou (1') de la revendication 1, comprenant l'utilisation, comme produit de départ, d'un diol de cyclohexène représenté par la formule suivante : 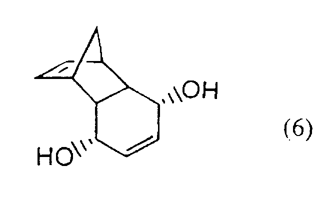 et la soumission du diol de cyclohexène à une transestérification en une position de façon sélective pour obtenir le dérivé optiquement actif désiré de cyclohexène.

5. Un procédé selon la revendication 4, dans lequel le diol de cyclohexène est transestérifié en présence d'une lipase pour obtenir le dérivé optiquement actif.

6. Un procédé selon la revendication 4, dans lequel le diol de cyclohexène est transestérifié non sélectivement et le diester est ensuite hydrolysé sélectivement en présence d'une lipase.

7. Un procédé pour produire un dérivé désiré de cyclohexénone optiquement actif, représenté par la formule suivante : 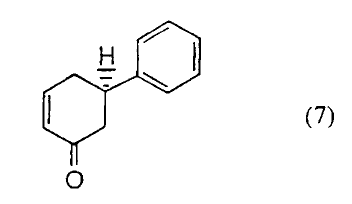 ou 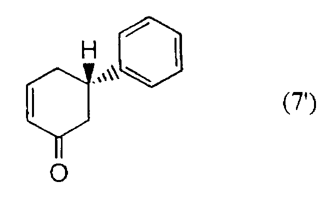 comprenant l'utilisation, comme produit de départ, d'un premier dérivé de cyclohexénone optiquement actif représenté par la formule (2) ou (2') selon la revendication 2, la réaction du premier dérivé par désacyloxylation pour obtenir un second dérivé de cyclohexénone optiquement actif représenté par la formule (3) ou (3') selon la revendication 3, l'isomérisation du second dérivé pour obtenir un troisième dérivé de cyclohexénone optiquement actif représenté par la formule (4) ou (4'), la réaction du troisième dérivé avec un réactif de Grignard pour obtenir un dérivé de cyclohexanone optiquement actif représenté par la formule (5) ou (5') et la réaction du dérivé de cyclohexanone par une réaction retro Diels-Alder pour obtenir le dérivé désiré de cyclohexénone optiquement actif.

8. Un procédé pour produire un dérivé de cyclohexène optiquement actif représenté par la formule (1) ou (1') selon la revendication 1, comprenant l'utilisation, comme produit de départ, d'un dérivé de diacyloxy cyclohexène représenté par la formule suivante (8), où R et R' sont identiques ou différents et chacun représente un groupe alkyle : 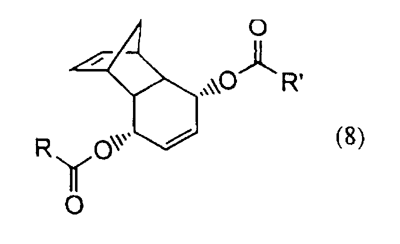 et la réaction du dérivé de diacyloxy cyclohexène, en présence de lipase par hydrolyse stéréosélective pour obtenir le dérivé désiré de cyclohexène optiquement actif.
